# EUROPEAN PATENT APPLICATION

(11) **EP 1 847 612 A1**
(43) Date of publication of application: **24.10.2007**
(21) Application number: 07002708.1
(22) Date of filing: 09.08.2002
(51) Int. Cl.: C12N 15/67, C07K 16/02, C07K 16/18, C12N 5/10

(54) **Expression of modified antibodies in avian cells**

(30) Priority: 10.08.2001 GB 0119497
(62) Divisional of application: 02755143.1
(71) Applicant: Viragen, Inc., Plantation, FL 33324 (US)
(72) Inventor: Jervis, Karen, Penicuik, Midlothian EH25 0PZ (GB); Robertson, Colin, Penicuik, Midlothian EH25 0PZ (GB); Conner, Joe, School of Biol. & Biomedical Science, Glasgow G4 0BA (GB); Stimson, William, Glasgow G4 0NR (GB)
(74) Representative: Stark, Gordon Drummond

(57) **Abstract**

The present invention relates to construct and methods which allow the expression of immunoglobulins or functional fragments thereof which have been altered so that they are humanised. The expression of the immunoglobulins or fragments thereof takes place in avian cells, and the constructs used have been altered such that the expression levels in avian cells are higher than what would have been expected by simply using a humanised construct. The alterations are based on changing codons so that each amino acid of the codon that is used is the one which is most often found in avians.

## Description

The present invention relates to the expression of immunoglobulins, or more specifically, antibodies which have been altered so that the antibody is 'humanised', in avian cells. The antibody expression may occur either *in vivo* or *in vitro.* In the following document, the terms immunoglobulin and antibody are used interchangeably.

Antibodies are proteins of the immunoglobulin class which are produced on exposure to an antigen. The antibody produced recognises that antigen, binding selectively to it. There are five classes of immunoglobulin and the following text relates primarily to antibodies of the class IgG, although the other classes: IgA, IgD, IgY, IgE and IgM are also included. An antibody molecule is made up of two identical heavy chains linked by disulphide bonds and two identical light chains. The biological effector functions of an antibody molecule derive from the properties of a constant region, which is identical for antibodies of all specificities within a particular class. It is the variable region that contains the site/s which allows binding to a particular epitope and there is a variable domain at the end of each of the heavy and light chains. These variable domains are followed by a number of constant domains. Thus, the binding of an antibody to an antigen occurs through interactions of the variable domains of each pair of heavy and light chains. Specifically, binding occurs in the areas of the variable regions where there is most variability. These regions are known as hyper variable regions or complementarity determining regions (CDRs).

Generally, with the exception of vaccinations, antibodies are generated from a non-human source such as a mouse. When used in human therapeutic applications, such antibodies are usually recognised as foreign by the immune system. This results in human anti-mouse antibodies being produced, which may reduce the therapeutic effect of the initial antibody or produce undesirable side effects. Techniques have been developed which allow the base of murine antibodies plus those of other species to be manipulated in a way that the original antigen specificity is retained, but all the non-essential parts of the immunoglobulin sequence are replaced with the equivalent human derived sequence. This is known as 'humanising' an antibody. By using humanised antibodies, immunogenic responses are largely or highly avoided and effector functions improved.

However, even when an immunoglobulin sequence is humanised there are still problems with the glycosylation pattern of immunoglobulins for use in humans which have not been produced in humans. IgG contains a conserved N-glycosylation site located within the CH2 domain of each heavy chain. The glycosylation pattern of immunoglobulins is highly heterogeneous and has been shown to have a significant effect on the biological, pharmacological and physiochemical properties of the immunoglobulin and include stability of the antibody and half-life, tolerance in patient treatment and interactions with complement components and other lg receptors. Alterations in the glycosylation pattern of IgG has been linked to symptoms of rheumatoid arthritis. The oligosaccharides are produced in the Golgi apparatus of the cellular interior and is regulated largely by the glycosyltransferases present in this organelle.

Currently, antibodies for therapeutic applications are being produced in a variety of cell lines and transgenically, but many of these systems are not particularly suitable in terms of glycosylation. The same antibody produced in different cell lines and animals may therefore be afforded different characteristics which may result in differing functions and pharmacokinetics. The expression of glycosyltransferases differs with different cell types, with the result that the glycosylation pattern of the protein produced differs from that produced by other cell types. In particular, it has been noted that non-human mammalian cell lines, for example hamster sell lines, show markedly different glycosylation patterns to humans and therefore are likely to cause problems with immunogenicity. It is known that normal Chinese Hamster Ovary (CHO) cells, which are the standard used in the industry for the manufacture of recombinant proteins, do not express the enzyme N-acetylglucosaminyltransferase-III (GlcNAcT-III), which has the role of synthesising carbohydrates which contain GlcNAc (Campbell & Stanley, 1984). Since GlcNAc is expressed in human immunoglobulin G, it is known that human proteins expressed in CHO cells are not glycosylated in this manner. It is also interesting to note that chicken IgG's also possess this oligosaccharide. It has been found by experts in the area, such as that reported by Raju et al. 2002, that the glycosylation patterns of avian cells are far more similar than non-human mammalian glycosylation patterns to human glycosylation patterns. It therefore can be seen that it would be highly advantageous to be able to utilise chicken cells as a mode of production for humanised recombinant proteins, compared to production of such proteins in the standard mammalian cells.

US Patent No US5225539 entitled "Recombinant Altered Antibodies and Method of Making Altered Antibodies" describes a method of replacing the complementarity determining regions of the heavy or light chain variable domains of the receiving antibody with the corresponding complementarity determining regions of a different antibody with a different specificity. This method, known as 'CDR grafting' is not the only method to produce humanised antibodies, bur is the most well known to those knowledgeable in the field. The present invention relates to antibodies which have been manipulated in any manner which has the result of producing an antibody which is more human-like in sequence than the wild-type sequence.

However, although Patent No US5225539 describes a general method which allows altered antibodies to be produced, the methods described are directed towards production of the altered antibodies in mammalian cells and does not envisage the problems and advantages that arise when producing antibodies in avian cells using this technology.

While the methodology described in Winters' Patent US5225539 is extremely useful for the production of CDR grafted antibodies in mammalian cells, it can be seen that it would also be useful to be able to easily produce such CDR grafted and humanised antibodies in avian cells. The production of such antibodies in avian cells would have a significant advantage over the production in mammalian cells if they are to be used as a human therapeutic. This is because the glycosylation pattern of human antibodies is more similar to avian antibodies than the glycosylation of mammalian antibodies. As previously mentioned, it is known that the glycosylation pattern can have a significant effect on the bioactivity, immunogenicity and therefore tolerance to the treatment and also the pharmacokinetics of the antibody itself, and therefore it would be extremely useful to produce antibodies for human use of which the glycosylation pattern is close to that of human antibodies.

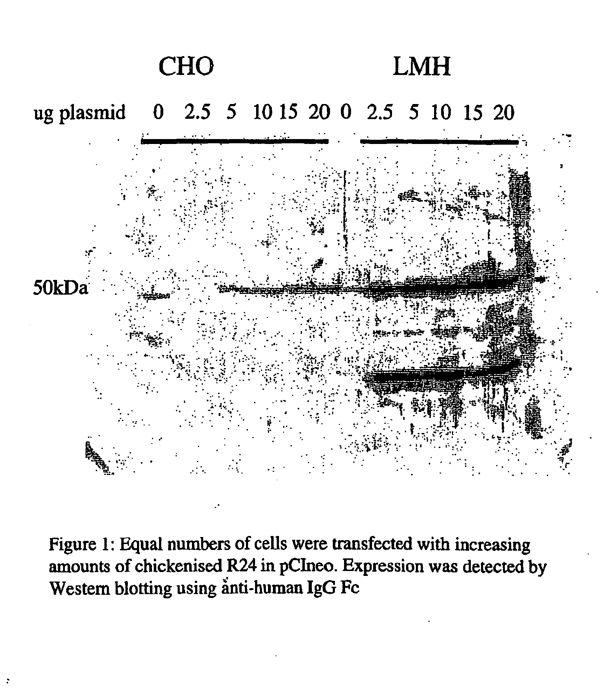

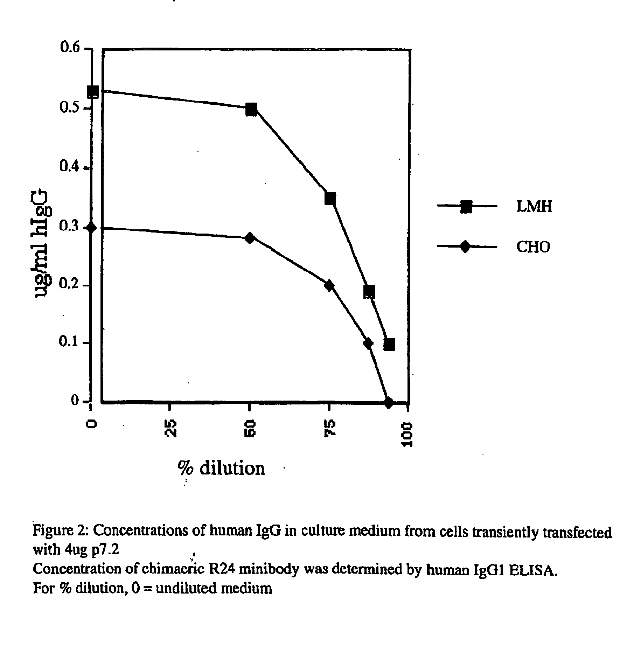

It is also known that the yield of proteins produced in non-avian transgenic animals, can be limited. It is common practice to have to alter the culture conditions or use expression vectors in order to obtain commercially viable expression levels. The inventors have shown that the expression of such proteins, especially immunoglobulins and fragments thereof, in avian cells is in general, higher than that observed in non-avian animals. It can therefore be seen that it would be useful and more time and cost-effective to produce antibodies for human therapeutic use in avian cells. Figure 1 shows the result of a Western blot to compare the expression levels of the R24 protein in Chicken Hepatocellular (LMH) cells, as compared to expression in Chinese Hamster Ovary (CHO) cells. Figure 2 illustrates a comparison between R24 protein produced in IMH and CHO cells, analysed by human IgG1 ELISA. It can be seen that there is a significantly higher level of protein produced in the LMH cells as compared to the CHO cells. The inventors believe that this is due to certain differences between the translational machinery of the cell types in that the LMH cells are more efficient in post-translationally modifying the protein, than mammalian cells. This belief is emphasised when the RNA message produced by both cell types is analysed by PCR gel and the levels produced by both types are similar.

One of the major problems of producing modified antibodies in avian cells is that the codon usage in avians differs from that in humans. Therefore, the methodology described in US Patent No 5225539 is not sufficient to allow the making of altered antibodies in avian cells efficiently.

There is also the problem of actually producing that which is coded for in a genetic construct in avian cells. US Patent No US4816397 describes methods of producing multi-chain polypeptides or proteins generally, however again the methods are discussed mainly with regard to mammalian cells and do not address the problems which occur when producing multi-chain polypeptides, such as antibodies, in avian cells. It should be noted that there are a number of Patents and Patent Applications which address the problem of producing basic single chain proteins in avian cells, however in these cases they do not refer to the production of complex multi-chain polypeptides, such as antibodies, which pose their own problems.

It can therefore be seen that it would be beneficial to provide a method of producing and expressing modified antibodies which have similar glycosylation patterns to those naturally produced in humans or more similar than the glycosylation patterns obtained from antibodies from culture in mammalian cells.

It can also be seen that it would beneficial to provide a method of producing and expressing modified antibodies in avian cells, as specifically producing and expressing humanised antibodies in avian cells, so that they can be used as human therapeutics.

It would also be extremely useful if the expression of the modified antibodies could be specifically in the egg of a genetically modified avian, so that the antibody can easily be collected and purified.

It is therefore a first object of the present invention to provide antibodies which have a glycosylation pattern which is more similar to naturally occurring human glycosylation patterns than those antibodies produced in mammalian cells.

It is a further object of the present invention to provide a method of expressing such humanised antibodies in avian cells *in vitro* and *in vivo.*

It is a further object of the present invention to provide a method of expressing humanised antibodies in avian cells which are known to produce higher yields than observed in non-avian cell systems.

It is a further object of the present, invention to provide a method of expressing humanised antibodies in avian cells *in vitro* and *in vivo.*

It is a yet further object of the present invention to provide antibodies for therapeutic use, which have glycosylation patterns which are more similar to those naturally occurring in humans, than the glycosylation patterns observed in antibodies produced in mammalian cells.

A still further object of the present invention is to provide a construct which can be delivered into avian cells, which will allow the production of antibodies or humanised antibodies.

A final object of the present invention is to provide a method of expressing humanised antibodies in avian cells, so that they are specifically produced in the egg white or egg yolk of an avian.

According to a first aspect of the present invention, there is provided a DNA construct which when transfected into an avian cell will allow the production of an antibody molecule or functional fragment of said molecule, and which comprises at least one sequence which comprises the variable domain of an immunoglobulin heavy chain and at least one sequence which comprises the variable domain of an immunoglobulin light chain, and wherein the DNA construct is based on a non-avian sequence and one or more of the codons in the DNA construct have been altered such that for the amino acid being encoded, the codon used is that which most frequently appears in avians.

Preferably, the construct also contains an avian signal peptide sequence.

Preferably the construct is cloned into a viral vector such as a lentivirus vector.

Most preferably, the avian signal peptide sequence is a signal peptide sequence from an egg white protein such as lysozyme, ovalbumin, ovatransferrin or ovamucoid.

Most preferably, the construct also includes immunoglobulin constant regions for dimerisation and recruitment of effector functions.

Most preferably, the immunoglobulin constant regions are CH2 and CH3 of any IgG class.

Still more preferably, the immunoglobulin constant regions are human constant regions in order to provide a humanised antibody.

Preferably, the construct may be transfected into an avian cell using lipofection.

Alternatively, the construct is transfected into an avian cell using electroporation.

A further option is that the construct may be directly injected into the nucleus of an avian, into the germinal disc of an oocyte.

Preferably, codon usage in the construct is maximised for those codons most frequently appearing in avians. That is, each codon is altered so that it still codes for the same amino acid, but uses the codon most often found to code for that amino acid in avians.

According to a second aspect of the present invention, there is provided an avian cell, containing the construct of the first aspect, which expresses an immunoglobulin molecule or functional fragment of said molecule.

Preferably the expressed immunoglobulin molecule or fragment thereof shows an avian glycosylation pattern.

Preferably the immunoglobulin or fragment thereof is expressed at a higher expression level than a standard human construct or humanised construct.

According to a third aspect of the present invention, there is provided a method for producing avian cells capable of expressing an immunoglobulin molecule or functional fragment of said molecule, comprising transfecting an avian cell with the DNA construct of the first aspect.

Preferably the avian cell is a chicken cell, but may also be duck, turkey, quail, or ostrich.

According to a fourth aspect of the present invention there is provided an immunoglobulin or functional fragment thereof produced using the method of the third aspect.

According to a fifth aspect of the present invention there is provided a transgenic avian, which expresses the construct of the first aspect.

Preferably the antibody molecule, or functional fragment of said molecule, that is coded for by the construct is expressed in an egg of the transgenic avian.

Most preferably the construct is expressed in the egg white.

Alternatively the construct is expressed in the egg yolk.

Preferably the immunoglobulin shows an avian glycosylation pattern.

The present invention will now be illustrated, by way of example only, with reference to the following Figures in which:
Figure 1 shows a Western blot showing the differences in protein expression between chicken and mammalian cells; and
Figure 2 is a graph illustrating the differences between protein expression levels in chicken and mammalian cells, as analysed by ELISA; and
Figure 3 is a table giving the frequency of codon usage in chickens.

And with reference to the following sequences in which;
SEQUENCE ID 1 is the sequence of human IgG Fc used for construction of chimeric and humanised minibodies;
and SEQUENCE ID 2 is the sequence of the chickenised IgG Fc DNA sequence.

SEQUENCE ID 3 is the nucleotide alignment of the original and chickenised human IgG Fc.

SEQUENCE ID 4 is the amino acid alignment of original and chickenised human IgG Fc.

SEQUENCE ID 5 shows the chickenised R24 nucleotide sequence.

SEQUENCE ID 6 shows the complete chickenised nucleotide sequence of the R24 chimeric minibody.

### Generating a Construct

In this example a construct is produced which allows a humanised murine R24 antibody to be produced in chickens.

The DNA encoding the single chain variable fragment is designed *in silico* so that it can then be directly synthesised using standard methods.

The starting sequences are human Vh and VI sequences which may be obtained from human IgM antibody. Vh and VI complementarity determining regions (CDRs) of another immunoglobulin (which in this case is the murine R24 immunoglobulin) are identified by standard methods (e.g. see Antibodies-Structure and Sequence at http://www.bioinf.org.uklabs) and the R24 CDRs are swapped directly into the human immunoglobulin framework.

The 3' end of the Vh DNA sequence is linked to the 5' end of the VI DNA sequence by a (Gly₄Ser)₃ peptide linker, as seen in SEQUENCE ID 1. Included at the 3'end of the VI sequence is a sequence encoding a Bam H1 restriction site. This gives the humanised R24 sequence 1. An IgG1 leader sequence is linked to the 5' end of Vh with the inclusion of an Eco R1 restriction site.

To provide the constant region of the immunoglobulin 2, human IgGl CH2/CH3 (Fc) DNA is then cloned by RT-PCR from RNA. The primers incorporate *Bam HI* and Sal 1 restriction sites and can be seen in SEQUENCE ID 2. The amplified DNA fragment is cloned directly following PCR using the PCR cloning vector pGEM-T (Promega). E coli DH5α cells are transformed with the ligated plasmid, plated out on amp selection media and colonies screened the following day by PCR with M13 vector primers.

Positive clones with the appropriately sized insert can then be selected and plasmid DNA can be prepared and inserts sequenced to confirm the presence of immunoglobulin constant region DNA. The insert from one positive DNA clone is removed by *Bam H1* / *Sal 1* digestion and ligated into pGEM 3Z (Promega), digested with the same restriction enzymes. After transformation and overnight growth on amp media, colonies are screened by PCR with M13 vector primers and plasmid prepared from one positive clone.

One µg of the R24 gene synthesis product 1 is digested with *EcoR1*/*BamH1* and ligated into plasmid hFc digested with the same enzymes. After transformation and overnight growth on amp media, colonies are screened by PCR with M13 vector primers and plasmids prepared from clones with the appropriately sized insert.

The entire insert DNA is then removed from pGEM3Z by digestion with the restriction enzymes *EcoR1*/ *Sal1* and ligated into the mammalian expression vector pCIneo (Promega) digested with the same enzymes. After transformation and overnight growth on amp media, colonies are screened by restriction digestion (*EcoR1*/*Sal1*) of plasmid preparations. Plasmids may be sequenced to confirm the presence of the minibody genes.

The insert can then be used to form a construct for insertion into an avian cell. The insert, comprising the Vh/VI CDRs transplanted into a human immunoglobulin framework 1 along with immunoglobulin constant domains 2 is removed from the pCIneo-vector by BgIII/Sfil digestion. The fragment that is gained by this digestion consists of;
a promoter/enhancer 6,
an intron 4,
the minibody which comprises the R24 variable regions 1 and the CH2 and CH3 constant regions 2, and
a poly A tail 3

In order for the construct to be suitable for expression in an avian cell, the immunoglobulin leader sequence is exchanged for an avian specific sequence such as the lysozyme signal peptide sequence 5. Also, both the R24 variable section coding sequence 1 and the CH2/CH3 constant region coding sequence 2 are chickenised.

### 'Chickenising' a Construct

Chickenising is defined as the alteration of codon usage such that it is maximised for those codons most frequently used in chickens. For expression in transgenic chickens the codons of. constructs are optimised for most frequent codon usage in chickens. However, it can be seen that the optimisation could be for the most frequent codon usage of any avian species.

### EXAMPLE

### Chickenising the human IgG Fc DNA sequence

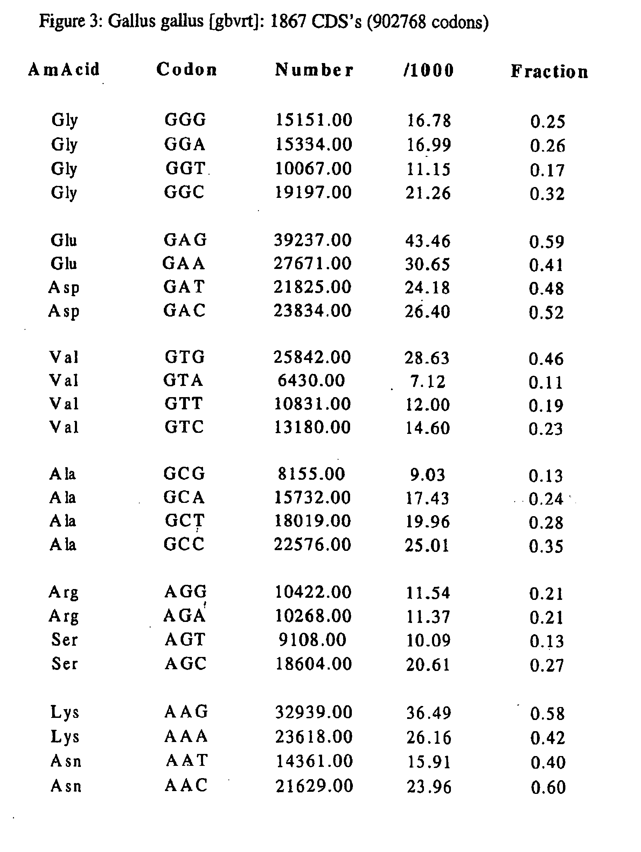

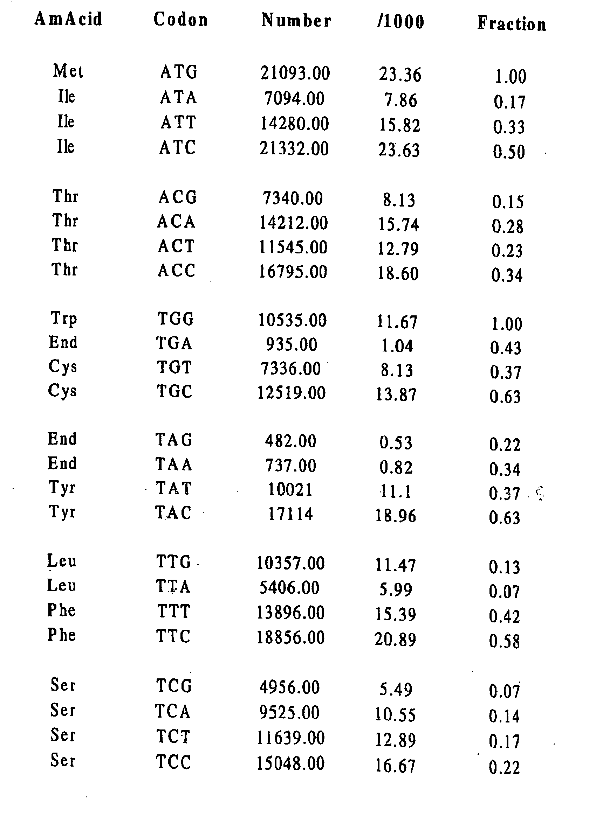

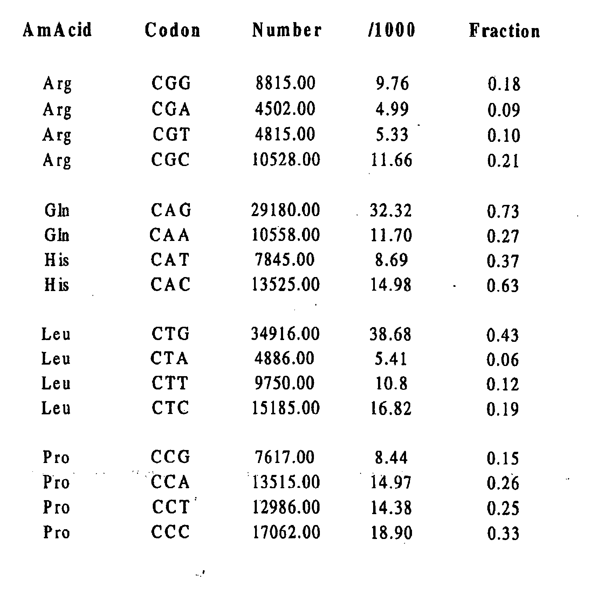

For expression in transgenic chickens the codons of the chimaeric and humanised minibody versions of R24 are optimised for most frequent codon usage in chickens (Gallus gallus). A table detailing frequency of codon usage was downloaded from http://www.kazusa.or.jp and is reproduced in Figure 3.

For an example of how chickenisation is carried out, it can be seen that the amino acid Valine is encoded by 4 different codons, GTG, GTA, GTT and GTC with GTG used most frequently in chickens (46% GTG, 11% GTA, 19% GTT and 23% GTC*). To chickenise the human IgG Fc DNA, all valine codons were converted to GTG. Lysine is encoded by two different codons, AAG and AAA, with AAG used most frequently in chickens (58% vs 42%). All AAA codons in the sequence were converted to AAG. Not all codons required alteration. For example, the two codons for aspartic acid, GAT and GAC are used with almost equally (48% vs 52%) and are not changed during the chickenisation.

Sequence ID 1 shows the codons for the original human IgG Fc DNA sequence. Sequence ID 2 shows the chickenised version of this. Sequence ID 3 shows an alignment of the nucleotide sequences, a dot indicates a sequence match and the missing dots show where the codons have been altered. Sequence ID 4 is an alignment of the amino acid sequences which show that despite the alterations to various codons the amino acids are still 100% identical. Sequence ID 5 and 6 show the chickenised R24 scFv sequence and complete chickenised R24 minibody respectively.
* figures as given in Figure 3 = 99%

### Inserting the Construct into an avian Cell

There are a number of possible methods that can be used for transfection of an avian cell with the construct. Transfection can either be transient or stable.

In transient transfection, supercoiled plasmid containing the gene of interest is introduced into the nucleus of the target cells at high copy number for short periods of time (usually 24-96 hrs). During transient transfections the DNA does not integrate into the cellular chromosomes.

For stable transfection either linear or plasmid DNA can be introduced into the target cells and will either integrate into the chromosomes or be maintained as a stable episome. Linear DNA is optimal for stable integration but is taken up less efficiently than supercoiled plasmid. Cells in which the DNA has integrated or is maintained as a stable episome can be distinguished by selectable markers. For transfections with pCIneo, the plasmid carries the neomycin phosphotransferase gene which confers resistance to aminoglycosides such as G418. Culturing cells in the presence of G418 selects for those that carry the integrated or episomal DNA.

A variety of methods are available for the introduction of DNA into mammalian cells and these include calcium phosphate coprecipitation (Graham, R. L. and van der Erb, AJA (1973) Virology 52, 456.) and electroporation (Andreason, G. L. and Evans, G.A. (1988) BioTechniques 6, 650 ; Shigikawa, K. and Dover, W. J., (1988) BioTechniques 6, 742) but these have largely been superseded by cationic liposome-mediated transfection (Felgner, J. et al (1993) J Tiss Cult Metho. 15,63). Other compounds known to mediate transfection of mammalian cells include lipopolyamines (Remy, J-S, Sirlin, C., Vierling, P and Behr, J-P. (1994) Bioconjugate Chem. 5, 647) and dendrimers (Haensler, J. and Szoka, FC (Jr) (1993) Bioconjugate Chem 4,372.).

Cationic liposomes, lipolyamines and dendrimers coat the DNA to be transfected and mediate its passage through the cell membrane. A variety of factors influence the efficiency of transfection and these include cell type, media type and presence of serum and antibiotics, amount and quality of plasmid DNA and cytotoxicity of transfection reagent. All of these usually have to be optimised for each cell type and plasmid construct. Alternatively, zygotic infection of the construct could also be used to incorporate the construct.

Alternatively the construct may be cloned into a viral vector such as a lentivirus vector and such vectors are commercially available.

Lentiviruses as vectors have been developed from slow retroviruses such as equine infectious anaemia virus (EIAV), feline immunodeficiency virus (FIV) or Human Immunodeficiency Virus (HIV). The significant advantage using a lentiviral vector is that the virus will infect cells that are not dividing, which is appropriate to certain cell types of-the present invention.

If the construct contains a promoter such as an egg white protein signal peptide, transgenic avians can then be produced which lay eggs with the antibody of interest in the egg white.

The chickenised construct may also be designed for insertion into a gene contained within a plasmid, for example it may be designed for insertion into a lysozyme gene contained within a plasmid. The ATG site on the lysozyme gene in the plasmid is destroyed by creating a Sal1 site so that the lysozyme protein is not expressed. The chickenised construct, which has its own ATG can then be cloned into the Sail site.

Various modifications may be made to the invention herein described, without departing from the scope thereof. For example, any appropriate immunoglobulin sequence may be used and any appropriate avian species may be used in place of chickens with the codon bias changing appropriately.

1
2
3 Sequence ID 1. The sequence of human IgG Fc used
4 for construction of chimaeric and humanised
5 minibodies.
6
7 AC CTT GCA GGA TCC GCA AGA CCC AAA TCT
8 TGT
9
10 GAC AAA ACT CAC ACA TGC CCA CCG TGC
11 CCA GCA
12
13 CCT GAA CTC CTG GGG GGA CCG TCA GTC
14 TTC CTC
15
16 TTC CCC CCA AAA CCC AAG GAC ACC CTC
17 ATG ATC
18
19 TCC CGG ACC CCT GAG GTC ACA TGC GTG
20 GTG GTG
21
22 GAC GTG AGC CAC GAA GAC CCT GAG GTC
23 AAG TTC
24
25 AAC TGG TAC GTG GAC GGC GTG GAG GTG
26 CAT AAT
27
28 GCC AAG ACA AAG CCG CGG GAG GAG CAG
29 TAC AAC
30
31 AGC ACG TAC CGG GTG GTC AGC GTC CTC
32 ACC GTC
33

1 CTG CAC CAG GAC TGG CTG AAT GGC AAG
2 GAG TAC
3
4 AAG TGC AAG GTC TCC AAC AAA GCC CTC
5 CCA GCC
6
7 CCC ATC GAG AAA ACC ATC TCC AAA GCC
8 AAA GGG
9
10 CAG CCC CGA GAA CCA CAG GTG TAC ACC
11 CTG CCC
12
13 CCA TCC CGG GAG GAG ATG ACC AAG AAC
14 CAG GTC
15
16 AGC CTG ACC TGC CTG GTC AAA GGC TTC
17 TAT CCC
18
19 AGC GAC ATC GCC GTG GAG TGG GAG AGC
20 AAT GGG
21
22 CAG CCG GAG AAC AAC TAC AAG ACC ACG
23 CCT CCC
24
25 GTG CTG GAC TCC GAC GGC TCC TTC TTC
26 CTC TAT
27
28 AGC AAG CTC ACC GTG GAC AAG AGC AGG
29 TGG CAG
30
31 CAG GGG AAC GTC TTC TCA TGC TCC GTG
32 ATG CAT
33

1 GAG GCT CTG CAC AAC CAC TAC ACG CAG
2 AAG AGC
3
4 CTC TCC CTG TCC CCG GGT AAA TGA TAA
5 GTC GAC
6
7 ACG TGA TC
8

1 Sequence ID 2. The chickenised human IgG Fc DNA
2 sequence.
3 Codon alterations are in red.
4
5
6 AC CTT GCA GGA TCC GCC AGA CCC AAG TC
7 TGC
8
9 GAC AAG ACC CAC ACA TGC CCA CCC TGC
10 CCA GCC
11
12 CCC GAG CTG CTG GGG GGA CCC TCC GTG
13 TTC CTG
14
15 TTC CCC CCA AAG CCC AAG GAC ACC CTG
16 ATG ATC
17
18 TCC CGC ACC CCC GAG GTG ACA TGC GTG
19 GTG GTG
20
21 GAC GTG AGC CAC GAG GAC CCC GAG GTG
22 AAG TTC
23
24 AAC TGG TAC GTG GAC GGC GTG GAG GTG
25 CAC AAC
26
27 GCC AAG ACA AAG CCC CGC GAG GAG CAG
28 TAC AAC
29
30 AGC ACC TAC CGC GTG GTG AGC GTG CTG
31 ACC GTG
32
33 CTG CAC CAG GAC TGG CTG AAC GGC AAG
34 GAG TAC

1
2 AAG TGC AAG GTG TCC AAC AAG GCC CTG
3 CCA GCC
4
5 CCC ATC GAG AAG ACC ATC TCC AAG GCC
6 AAG GGG
7
8 CAG CCC CGC GAG CCA CAG GTG TAC ACC
9 CTG CCC
10
11 CCA TCC CGC GAG GAG ATG ACC AAG AAC
12 CAG GTG
13
14 AGC CTG ACC TGC CTG GTG AAG GGC TTC
15 TAC CCC
16
17 AGC GAC ATC GCC GTG GAG TGG GAG AGC
18 AAC GGG
19
20 CAG CCC GAG AAC AAC TAC AAG ACC ACC
21 CCC CCC
22
23 GTG CTG GAC TCC GAC GGC TCC TTC TTC
24 CTG TAC
25
26 AGC AAG CTG ACC GTG GAC AAG AGC AGG
27 TGG CAG
28
29 CAG GGG AAC GTG TTC TCC TGC TCC GTG
30 ATG CAC
31
32 GAG GCC CTG CAC AAC CAC TAC ACC CAG
33 AAG AGC

1
2 CTC TCC CTG TCC CCC GGC AAG TGA TAA
3 GTG GAC
4
5 ACC TGA TC
6

1
2 Sequence ID 3. Nucleotide alignment of the
3 original (upper) and chickenised (lower) human
4 IgG Fc.
5
6 10 20 30 40 50 60
7 70
8 | | | | | |
9 |
10 ACCTTGCAGGATCCGCAAGACCCAAATCTTGTGACAAAACTCACACATGCCCACCGTGCCCAGCACCTG
11 AACTCCTG
12 *********** ***** ******** ** ** ***** ** ************** ******** **
13 ** ** ***
14 ACCTTGCAGGATCCGCCAGACCCAAGTCCTGCGACAAGACCCACACATGCCCACCCTGCCCAGCCCCCG
15 AGCTGCTG
16 | | | | | |
17 |
18 10 20 30 40 50 60
19 70
20
21
22
23 80 90 100 110 120 130 140
24 150
25 | | | | | | |
26 |
27 GGGGGACCGTCAGTCTTCCTCTTCCCCCCAAAACCCAAGGACACCCTCATGATCTCCCGGACCCCTGAG
28 GTCACATG
29 ******** ** ** ***** *********** ************* ********** *****
30 ***** *****
31 GGGGGACCCTCCGTGTTCCTGTTCCCCCCAAAGCCCAAGGACACCCTGATGATCTCCCGCACCCCCGAG
32 CTGACATG
33 | | | | | | |
34 |
35 80 90 100 110 120 130 140
36 150
37
38
39
40 160 170 180 190 200 210 220
41 230
42 | | | | | | |
43 |
44 CGTGGTGGTGGACGTGAGCCACGAAGACCCTGAGGTCAAGTTCAACTGGTACGTGGACGGCGTGGAGGT
45 GCATAATG
46 ********************** ***** *****
47 ************************************** ** *
48 CGTGGTGGTGGACGTGAGCCACGAGGACCCCGAGGTGAAGTTCAACTGGTACGTGGACGGCGTGGAGGT
49 GCACAACG
50 | | | | | | |
51 |
52 160 170 180 190 200 210 220
53 230
54
55
56

1 240 250 260 270 280 290
2 300
3 | | | | | | |
4 CCAAGACAAAGCCGCGGGAGGAGCAGTACAACAGCACGTACCGGGTCGTCAGCGTCCTCACCGTCCTGC
5 ACCAGGAC
6 ••••••••••••• •• •••••••••••••••••••• ••••• ••••• ••••• •• •••••
7 ••••••••••••
8 CCAAGACAAAGCCCCGCGAGGAGCAGTACAACAGCACCTACCGCGTGGTGAGCGTGCTGACCGTGCTGC
9 ACCAGGAC
10 | | | | | | |
11 240 250 260 270 280 290
12 300
13
14
15
16 310 320 330 340 350 360 370
17 380
18 | | | | | | |
19 |
20 TGGCTGAATGGCAAGGAGTACAAGTGCAAGGTCTCCAACAAAGCCCTCCCAGCCCCCATCGAGAAAACC
21 ATCTCCAA
22 ••••••• •••••••••••••••••••• •••••••• ••••• ••••••••••••••••
23 •••••••••••
24 TGGCTGAACGGCAAGGAGTACAAGTGCAAGGTGTCCAACAAGGCCCTGCCAGCCCCCATCGAGAAGACC
25 ATCTCCAA
26 | | | | | | |
27 |
28 310 320 330 340 350 360 370
29 380
30
31
32
33 390 400 410 420 430 440 450
34 460
35 | | | | | | |
36 |
37 AGCCAAAGGGCAGCCCCGAGAACCACAGGTGTACACCCTGCCCCCATCCCGGGAGGAGATGACCAAGAA
38 CCAGGTCA
39 ••••• •••••••••• •• ••••••••••••••••••••••••••••
40 ••••••••••••••••••••••• •
41 GGCCAAGGGGCAGCCCCGCGAGCCACAGGTGTACACCCTGCCCCCATCCCGCGAGGAGATGACCAAGAA
42 CCAGGTGA
43 | | | | | | |
44 |
45 390 400 410 420 430 440 450
46 460
47
48
49
50 470 480 490 500 510 520
51 530
52 | | | | | | |
53 GCCTGACCTGCCTGGTCAAAGGCTTCTATCCCAGCGACATCGCCGTGGAGTGGGAGAGCAATGGGCAGC
54 CGGAGAAC
55 •••••••••••••••• •• •••••••• ••••••••••••••••••••••••••••••••
56 •••••••• ••••••
57 GCCTGACCTGCCTGGTGAAGGGCTTCTACCCCAGCGACATCGCCGTGGAGTGGGAGAGCAACGGGCAGC
58 CCGAGAAC
59 | | | | | | |

1 470 480 490 500 510 520
2 530
3
4
5
6 540 550 560 570 580 590 600
7 610
8 | | | | | | |
9 |
10 AACTACAAGACCACGCCTCCCGTGCTGGACTCCGACGGCTCCTTCTTCCTCTATAGCAAGCTCACCGTG
11 GACAAGAG
12 ••••••••••••••• •• •••••••••••••••••••••••••••••••••••• •• ••••••••
13 ••••••••••••••
14 AACTACAAGACCACCCCCCCCGTGCTGGACTCCGACGGCTCCTTCTTCCTGTACAGCAAGCTGACCGTG
15 GACAAGAG
16 | | | | | | |
17 |
18 540 550 560 570 580 590 600
19 610
20
21
22
23 620 630 640 650 660 670 680
24 690
25 | | | | | | |
26 |
27 CAGGTGGCAGCAGGGGAACGTCTTCTCATGCTCCGTGATGCATGAGGCTCTGCACAACCACTACACGCA
28 GAAGACCC
29 •••••••••••••••••••• ••••• •••••••••••••• ••••• •••••••••••••••••
30 ••••••••••
31 CAGGTGGCAGCAGCCCAACGTGTTCTCCTGCTCGGTGATGCACGAGGCCCTGCACAACCACTACACCCA
32 GAAGAGCC
33 | | | | | | |
34 |
35 620 630 640 650 660 670 680
36 690
37
38
39
40 700 710 720 730
41 | | | |
42 TCTCCCTGTCCCCGGGTAAATGATAAGTCGACACGTGATC
43 •••••••••••••• •• •• •••••••• ••••• •••••
44 TCTCCCTGTCCCCCGGCAAGTGATAAGTGGACACCTGATC
45 | | | |
46 700 710 720 730
47
48
49
50

1
2 Sequence ID4. Amino acid alignment of original
3 (upper) and chickenised (lower) human IgG Fc.
4
5 10 20 30 40 50 60
6 70
7 | | | | | |
8 |
9 LAGSARPKSCDKTHTCPPCPAPELLGGPSVFLFPPKPKDTLMISRTPEVTCVVVDVSHEDPEVKFNWYV
10 DGVEVHNA
11 ......................................................................
12 ........
13 LAGSARPKSCDKTHTCPPCPAPELLGGPSVFLFPPKPKDTLMISRTPEVTCVVVDVSHEDPEVKFNWYV
14 DGVEVHNA
15 | | | | | |
16 |
17 10 20 30 40 50 60
18 70
19
20
21
22 80 90 100 110 120 130 140
23 150
24 | | | | | | |
25 |
26 KTKPREEQYNSTYRVVSVLTVLHQDWLNGKEYKCKVSNKALPAPIEKTISKAKGQPREPQVYTLPPSRE
27 EMTKNQVS
28 ......................................................................
29 ........
30 KTKPREEQYNSTYRVVSVLTVLHQDWLNGKEYKCKVSNKALPAPIEKTISKAKGQPREPQVYTLPPSRE
31 EMTKNQVS
32 | | | | | | |
33 |
34 80 90 100 110 120 130 140
35 150
36
37
38
39 160 170 180 190 200 210 220
40 230
41 | | | | | | |
42 |
43 LTCLVKGFYPSDIAVEWESNGQPENNYKTTPPVLDSDGSFFLYSKLTVDKSRWQQGNVFSCSVMHEALH
44 NHYTQKSL
45 ......................................................................
46 ........
47 LTCLVKGFYPSDIAVEWESNGQPENNYKTTPPVLDSDGSFFLYSKLTVDKSRWQQGNVFSCSVMHEALH
48 NHYTQKSL
49 | | | | | | |
50 |
51 160 170 180 190 200 210 220
52 230
53
54
55
56 240
57 |
58 SLSPGK--VDT

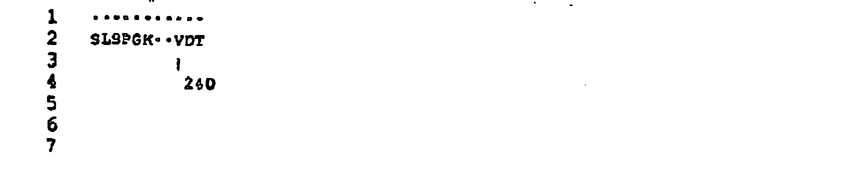

1
2
3 Sequence ID5. Chickenised R24 sequence. Altere
4 codons are shown in red. The underlined
5 nucleotides encode the lysozyme leader amino
6 acids
7
8 GGC CGG GTC GAC ATG AGG TCT TTG CTA
9 ATC TTG
10
11 GTG CTT TGC TTC CTG CCC CTG GCT GCT
12 CTG GGG
13
14 GAT GTG CAG CTG GTG GAG TCC GGG GGA
15 GGC CTG
16
17 GTG CAG CCC GGA GGG TCC CGC AAG CTC
18 TCC TGC
19
20 GCC GCC TCC GGA TTC ACC TTC AGC AAC
21 TTC GGA
22
23 ATG CAC TGG GTG CGC CAG GCC CCC GAG
24 AAG GGG
25
26 CTG GAG TGG GTG GGA TAC ATC AGC AGC
27 GGC GGC
28
29 AGC TCC ATC AAC TAC GCC GAC ACC GTG
30 AAG GGC
31
32 CGC TTC ACC ATC TCC AGA GAC AAC CCC
33 AAG AAC
34

1 ACC CTG TTC CTG CAG ATG ACC AGC CTG
2 AGG TCC
3
4 GAG GAC ACA GCC ATC TAC TAC TGC ACC
5 AGA GGG
6
7 GGA ACC GGG ACC AGA TCC CTG TAC TAC
8 TTC GAC
9
10 TAC TGG GGC CAG GGC GCC ACA CTG ATC
11 GTG TCC
12
13 TCC GGG GGA GGC GGC TCC GGG GGA GGC
14 GGC TCC
15
16 GGG GGA GGC GGC TCC GAT ATC CAG ATG
17 ACA CAG
18
19 ATC ACA TCC TCC CTG TCT GTG TCT CTG
20 GGA GAC
21
22 AGA GTG ATC ATC AGC TGC AGG GCT AGC
23 CAG GAC
24
25 ATC GGC AAT TTT CTG AAC TGG TAC CAG
26 CAG GAA
27
28 CCA GAT GGA TCT CTG AAG CTG CTG ATC
29 TAC TAC
30
31 ACA TCT AGA CTG CAG TCC GGA GTG CCA
32 TCC AGG
33

1 TTC AGC GGC TGG GGG TCT GGA ACA GAT
2 TAC TCT
3
4 CTG ACC ATT AGC AAC CTG GAG GAA GAG
5 GAT ATC
6
7 GCC ACC TTC TTC TGC CAG CAG GGC AAG
8 ACA CTG
9
10 CCC TAC ACC TTC GGA GGG GGG ACC AAG
11 CTG GAG
12
13 ATC AAG CGC GGA TCC GCC GCC G

1 Sequence ID6. The complete chickenised nucleotide
2 sequence of the R24 chimaeric minibody.
3
4 GGC CGG GTC GAC ATG AGG TCT TTG CTA
5 ATC TTG
6
7 GTG CTT TGC TTC CTG CCC CTG GCT GCT
8 CTG GGG
9
10 GAT GTG CAG CTG GTG GAG TCC GGG GGA
11 GGC CTG
12
13 GTG CAG CCC GGA GGG TCC CGC AAG CTC
14 TCC TGC
15
16 GCC GCC TCC GGA TTC ACC TTC AGC AAC
17 TTC GGA
18
19 ATG CAC TGG GTG CGC CAG GCC CCC GAG
20 AAG GGG
21
22 CTG GAG TGG GTG GGA TAC ATC AGC AGC
23 GGC GGC
24
25 AGC TCC ATC AAC TAC GCC GAC ACC GTG
26 AAG GGC
27
28 CGC TTC ACC ATC TCC AGA GAC AAC CCC
29 AAG AAC
30
31 ACC CTG TTC CTG CAG ATG ACC AGC CTG
32 AGG TCC
33

1 GAG GAC ACA GCC ATC TAC TAC TGC ACC
2 AGA GGG
3
4 GGA ACC GGG ACC AGA TCC CTG TAC TAC
5 TTC GAC
6
7 TAC TGG GGC CAG GGC GCC ACA CTG ATC
8 GTG TCC
9
10 TCC GGG GGA GGC GGC TCC GGG GGA GGC
11 GGC TCC
12
13 GGG GGA GGC GGC TCC GAT ATC CAG ATG
14 ACA CAG
15
16 ATC ACA TCC TCC CTG TCT GTG TCT CTG
17 GGA GAC
18
19 AGA GTG ATC ATC AGC TGC AGG GCT AGC
20 CAG GAC
21
22 ATC GGC AAT TTT CTG AAC TGG TAC CAG
23 CAG GAA
24
25 CCA GAT GGA TCT CTG AAG CTG CTG ATC
26 TAC TAC
27
28 ACA TCT AGA CTG CAG TCC GGA GTG CCA
29 TCC AGG
30
31 TTC AGC GGC TGG GGG TCT GGA ACA GAT
32 TAC TCT
33

1 CTG ACC ATT AGC AAC CTG GAG GAA GAG
2 GAT ATC
3
4 GCC ACC TTC TTC TGC CAG CAG GGC AAG
5 ACA CTG
6
7 CCC TAC ACC TTC GGA GGG GGG ACC AAG
8 CTG GAG
9
10 ATC AAG CGC GGA TCC GCC AGA CCC AAG
11 TCC TGC
12
13 GAC AAG ACC CAC ACA TGC CCA CCC TGC
14 CCA GCC
15
16 CCC GAG CTG CTG GGG GGA CCC TCC GTG
17 TTC CTG
18
19 TTC CCC CCA AAG CCC AAG GAC ACC CTG
20 ATG ATC
21
22 TCC CGC ACC CCC GAG GTG ACA TGC GTG
23 GTG GTG
24
25 GAC GTG AGC CAC GAG GAC CCC GAG GTG
26 AAG TTC
27
28 AAC TGG TAC GTG GAC GGC GTG GAG GTG
29 CAC AAC
30
31 GCC AAG ACA AAG CCC CGC GAG GAG CAG
32 TAC AAC
33

1 AGC ACC TAC CGC GTG GTG AGC GTG CTG
2 ACC GTG
3
4 CTG CAC CAG GAC TGG CTG AAC GGC AAG
5 GAG TAC
6
7 AAG TGC AAG GTG TCC AAC AAG GCC CTG
8 CCA GCC
9
10 CCC ATC GAG AAG ACC ATC TCC AAG GCC
11 AAG GGG
12
13 CAG CCC CGC GAG CCA CAG GTG TAC ACC
14 CTG CCC
15
16 CCA TCC CGC GAG GAG ATG ACC AAG AAC
17 CAG GTG
18
19 AGC CTG ACC TGC CTG GTG AAG GGC TTC
20 TAC CCC
21
22 AGC GAC ATC GCC GTG GAG TGG GAG AGC
23 AAC GGG
24
25 CAG CCC GAG AAC AAC TAC AAG ACC ACC
26 CCC CCC
27
28 GTG CTG GAC TCC GAC GGC TCC TTC TTC
29 CTG TAC
30
31 AGC AAG CTG ACC GTG GAC AAG AGC AGG
32 TGG CAG
33

1 CAG GGG AAC GTG TTC TCC TGC TCC GTG
2 ATG CAC
3
4 GAG GCC CTG CAC AAC CAC TAC ACC CAG
5 AAG AGC
6
7 CTC TCC CTG TCC CCC GGC AAG TGA TAA
8 GTC GAC
9
10 ACC TGA TC
11

## Claims

1. A method of increasing the expression of an immunoglobulin in a cell, wherein the method comprises the steps of:
- providing an avian cell,
- transfecting the avian cell with a lentivirus vector comprising a DNA construct which comprises at least one DNA sequence which encodes for the variable domain of an immunoglobulin heavy chain and at least one DNA sequence which encodes for the variable domain of an immunoglobulin light chain,
- expressing the immunoglobulin within the cell, and
- isolating the immunoglobulin from the cell.

2. A method as claimed in claim 1 wherein the DNA construct further contains an avian signal peptide sequence.

3. A method as claimed in claim 2 wherein the avian signal peptide is a signal peptide sequence from an egg white protein.

4. A method as claimed in claim 3 wherein the egg white protein is lysozyme, ovalbumin, ovatransferrin or ovamucoid.

5. A method as claimed in any one of claims 1 to 4 wherein the construct further sequence encoding for at least one immunoglobulin constant region.

6. A method as claimed in claim 5 wherein the immunoglobulin constant regions is CH2 and/or CH3.

7. A method as claimed in claim 5 or 6 wherein the at least one immunoglobulin constant region is derived from a human immunoglobulin.

8. A method for producing an avian cell which is capable of expressing an immunoglobulin molecule or functional fragment thereof the method comprising the steps of:
- transfecting the avian cell with a lentivirus vector comprising a DNA construct which comprises at least one DNA sequence which encodes for the variable domain of an immunoglobulin heavy chain and at least one DNA sequence which encodes for the variable domain of an immunoglobulin light chain.

9. A lentiviral vector for use in the expression of an immunoglobulin or fragment thereof in an avian cell, the vector comprising a DNA construct which comprises at least one DNA sequence which encodes for the variable domain of an immunoglobulin heavy chain and at least one DNA sequence which encodes for the variable domain of an immunoglobulin light chain.

10. A transgenic avian expressing the lentiviral vector of claim 9.

11. An antibody binding fragment encoded by the nucleotide sequence of SEQ ID NO:6.


### Annex to the application documents - subsequently filed sequences listing

SEQUENCE LISTING

<110>  Viragen Incorporated

<120>  Expression of Modified Antibodies in Avian Cells

<130>  P32549.EP.06/AMC/GST

<140>  EP07002708.1
<141>  2002-08-09

<150>  GB 0119497.6
<151>  2001-08-10

<160>  6

<170>  PatentIn version 3.3

<210>  1
<211>  733
<212>  DNA
<213>  Homo sapiens

<400>  1
accttgcagg atccgcaaga cccaaatctt gtgacaaaac tcacacatgc ccaccgtgcc      60

cagcacctga actcctgggg ggaccgtcag tcttcctctt ccccccaaaa cccaaggaca     120

ccctcatgat ctcccggacc cctgaggtca catgcgtggt ggtggacgtg agccacgaag     180

accctgaggt caagttcaac tggtacgtgg acggcgtgga ggtgcataat gccaagacaa     240

agccgcggga ggagcagtac aacagcacgt accgggtggt cagcgtcctc accgtcctgc     300

accaggactg gctgaatggc aaggagtaca agtgcaaggt ctccaacaaa gccctcccag     360

cccccatcga gaaaaccatc tccaaagcca aagggcagcc ccgagaacca caggtgtaca     420

ccctgccccc atcccgggag gagatgacca agaaccaggt cagcctgacc tgcctggtca     480

aaggcttcta tcccagcgac atcgccgtgg agtgggagag caatgggcag ccggagaaca     540

actacaagac cacgcctccc gtgctggact ccgacggctc cttcttcctc tatagcaagc     600

tcaccgtgga caagagcagg tggcagcagg ggaacgtctt ctcatgctcc gtgatgcatg     660

aggctctgca caaccactac acgcagaaga gcctctccct gtccccgggt aaatgataag     720

tcgacacgtg atc                                                        733


<210>  2
<211>  733
<212>  DNA
<213>  Artificial

<220>
<223>  chickenised IgG Fc

<400>  2
accttgcagg atccgccaga cccaagtcct gcgacaagac ccacacatgc ccaccctgcc      60

cagcccccga gctgctgggg ggaccctccg tgttcctgtt ccccccaaag cccaaggaca     120

ccctgatgat ctcccgcacc cccgaggtga catgcgtggt ggtggacgtg agccacgagg     180

accccgaggt gaagttcaac tggtacgtgg acggcgtgga ggtgcacaac gccaagacaa     240

agccccgcga ggagcagtac aacagcacct accgcgtggt gagcgtgctg accgtgctgc 300

accaggactg gctgaacggc aaggagtaca agtgcaaggt gtccaacaag gccctgccag 360

cccccatcga gaagaccatc tccaaggcca aggggcagcc ccgcgagcca caggtgtaca 420

ccctgccccc atcccgcgag gagatgacca agaaccaggt gagcctgacc tgcctggtga 480

agggcttcta ccccagcgac atcgccgtgg agtgggagag caacgggcag cccgagaaca 540

actacaagac cacccccccc gtgctggact ccgacggctc cttcttcctg tacagcaagc 600

tgaccgtgga caagagcagg tggcagcagg ggaacgtgtt ctcctgctcc gtgatgcacg 660

aggccctgca caaccactac acccagaaga gcctctccct gtcccccggc aagtgataag 720

tggacacctg atc 733

<210> 3
<211> 242
<212> PRT
<213> Homo sapiens

<220>
<221> misc_feature
<222> (238)..(239)
<223> Xaa can be any naturally occurring amino acid

<400> 3

Leu Ala Gly Ser Ala Arg Pro Lys Ser Cys Asp Lys Thr His Thr Cys
1               5                   10                  15

Pro Pro Cys Pro Ala Pro Glu Leu Leu Gly Gly Pro Ser Val Phe Leu
            20                  25                  30

Phe Pro Pro Lys Pro Lys Asp Thr Leu Met Ile Ser Arg Thr Pro Glu
        35                  40                  45

Val Thr Cys Val Val Val Asp Val Ser His Glu Asp Pro Glu Val Lys
    50                  55                  60

Phe Asn Trp Tyr Val Asp Gly Val Glu Val His Asn Ala Lys Thr Lys
65                  70                  75                  80

Pro Arg Glu Glu Gln Tyr Asn Ser Thr Tyr Arg Val Val Ser Val Leu
                85                  90                  95

Thr Val Leu His Gln Asp Trp Leu Asn Gly Lys Glu Tyr Lys Cys Lys
            100                 105                 110

Val Ser Asn Lys Ala Leu Pro Ala Pro Ile Glu Lys Thr Ile Ser Lys
        115                 120                 125

Ala Lys Gly Gln Pro Arg Glu Pro Gln Val Tyr Thr Leu Pro Pro Ser
    130                 135                 140

Arg Glu Glu Met Thr Lys Asn Gln Val Ser Leu Thr Cys Leu Val Lys
145                 150                 155                 160

Gly Phe Tyr Pro Ser Asp Ile Ala Val Glu Trp Glu Ser Asn Gly Gln
                165                 170                 175

Pro Glu Asn Asn Tyr Lys Thr Thr Pro Pro Val Leu Asp Ser Asp Gly
            180                 185                 190

Ser Phe Phe Leu Tyr Ser Lys Leu Thr Val Asp Lys Ser Arg Trp Gln
        195                 200                 205

Gln Gly Asn Val Phe Ser Cys Ser Val Met His Glu Ala Leu His Asn
    210                 215                 220

His Tyr Thr Gln Lys Ser Leu Ser Leu Ser Pro Gly Lys Xaa Xaa Val
225                 230                 235                 240

Asp Thr

<210>  4
<211>  242
<212>  PRT
<213>  Artificial

<220>
<223>  chickenised human IgG Fc

<220>
<221>  misc_feature
<222>  (238)..(239)
<223>  Xaa can be any naturally occurring amino acid

<400>  4

Leu Ala Gly Ser Ala Arg Pro Lys Ser Cys Asp Lys Thr His Thr Cys
1               5                   10                  15

Pro Pro Cys Pro Ala Pro Glu Leu Leu Gly Gly Pro Ser Val Phe Leu
            20                  25                  30

Phe Pro Pro Lys Pro Lys Asp Thr Leu Met Ile Ser Arg Thr Pro Glu
        35                  40                  45

Val Thr Cys Val Val Val Asp Val Ser His Glu Asp Pro Glu Val Lys
    50                  55                  60

Phe Asn Trp Tyr Val Asp Gly Val Glu Val His Asn Ala Lys Thr Lys
65                  70                  75                  80

Pro Arg Glu Glu Gln Tyr Asn Ser Thr Tyr Arg Val Val Ser Val Leu
                85                  90                  95

Thr Val Leu His Gln Asp Trp Leu Asn Gly Lys Glu Tyr Lys Cys Lys
            100                 105                 110

Val Ser Asn Lys Ala Leu Pro Ala Pro Ile Glu Lys Thr Ile Ser Lys
        115                 120                 125

Ala Lys Gly Gln Pro Arg Glu Pro Gln Val Tyr Thr Leu Pro Pro Ser
    130                 135                 140

Arg Glu Glu Met Thr Lys Asn Gln Val Ser Leu Thr Cys Leu Val Lys
145                 150                 155                 160

Gly Phe Tyr Pro Ser Asp Ile Ala Val Glu Trp Glu Ser Asn Gly Gln
                165                 170                 175

Pro Glu Asn Asn Tyr Lys Thr Thr Pro Pro Val Leu Asp Ser Asp Gly
            180                 185                 190

Ser Phe Phe Leu Tyr Ser Lys Leu Thr Val Asp Lys Ser Arg Trp Gln
        195                 200                 205

Gln Gly Asn Val Phe Ser Cys Ser Val Met His Glu Ala Leu His Asn
    210                 215                 220

His Tyr Thr Gln Lys Ser Leu Ser Leu Ser Pro Gly Lys Xaa Xaa Val
225                 230                 235                 240

Asp Thr


<210>  5
<211>  814
<212>  DNA
<213>  Artificial

<220>
<223>  chickenised R24 scFv sequence


<220>
<221>  lysozyme_leader
<222>  (13)..(66)

<400>  5
ggccgggtcg acatgaggtc tttgctaatc ttggtgcttt gcttcctgcc cctggctgct      60

ctgggggatg tgcagctggt ggagtccggg ggaggcctgg tgcagcccgg agggtcccgc     120

aagctctcct gcgccgcctc cggattcacc ttcagcaact tcggaatgca ctgggtgcgc     180

caggcccccg agaaggggct ggagtgggtg ggatacatca gcagcggcgg cagctccatc     240

aactacgccg acaccgtgaa gggccgcttc accatctcca gagacaaccc caagaacacc     300

ctgttcctgc agatgaccag cctgaggtcc gaggacacag ccatctacta ctgcaccaga     360

gggggaaccg ggaccagatc cctgtactac ttcgactact ggggccaggg cgccacactg     420

atcgtgtcct ccgggggagg cggctccggg ggaggcggct ccgggggagg cggctccgat 480

atccagatga cacagatcac aatcggcaat tttctgaact ggtaccagca ggaaccagat 540

ggatctctga agctgctgat ctactacaca tctagactgc agtccggagt gccatccagg 600

ttcagcggct gggggtctgg aacagattac tctctgacca ttagcaacct ggaggaagag 660

gatatctcct ccctgtctgt gtctctggga gacagagtga tcatcagctg cagggctagc 720

caggacgcca ccttcttctg ccagcagggc aagacactgc cctacacctt cggagggggg 780

accaagctgg agatcaagcg cggatccgcc gccg 814

<210> 6
<211> 1526
<212> DNA
<213> Artificial

<220>
<223> complete chickenised R24 minibody sequence

<400> 6
ggccgggtcg acatgaggtc tttgctaatc ttggtgcttt gcttcctgcc cctggctgct 60

ctgggggatg tgcagctggt ggagtccggg ggaggcctgg tgcagcccgg agggtcccgc 120

aagctctcct gcgccgcctc cggattcacc ttcagcaact tcggaatgca ctgggtgcgc 180

caggcccccg agaaggggct ggagtgggtg gcatacatca gcagcggcgg cagctccatc 240

aactacgccg acaccgtgaa gggccgcttc accatctcca gagacaaccc caagaacacc 300

ctgttcctgc agatgaccag cctgaggtcc gaggacacag ccatctacta ctgcaccaga 360

gggggaaccg ggaccagatc cctgtactac ttcgactact ggggccaggg cgccacactg 420

atcgtgtcct ccgggggagg cggctccggg ggaggcggct ccgggggagg cggctccgat 480

atccagatga cacagatcac atcctccctg tctgtgtctc tgggagacag agtgatcatc 540

agctgcaggg ctagccagga catcggcaat tttctgaact ggtaccagca ggaaccagat 600

ggatctctga agctgctgat ctactacaca tctagactgc agtccggagt gccatccagg 660

ttcagcggct gggggtctgg aacagattac tctctgacca ttagcaacct ggaggaagag 720

gatatcgcca ccttcttctg ccagcagggc aagacactgc cctacacctt cggagggggg 780

accaagctgg agatcaagcg cggatccgcc agacccaagt cctgcgacaa gacccacaca 840

tgcccaccct gcccagcccc cgagctgctg gggggaccct ccgtgttcct gttcccccca 900

aagcccaagg acaccctgat gatctcccgc acccccgagg tgacatgcgt ggtggtggac 960

gtgagccacg aggaccccga ggtgaagttc aactggtacg tggacggcgt ggaggtgcac 1020

aacgccaaga caaagccccg cgaggagcag tacaacagca cctaccgcgt ggtgagcgtg 1080

ctgaccgtgc tgcaccagga ctggctgaac ggcaaggagt acaagtgcaa ggtgtccaac 1140

aaggccctgc cagcccccat cgagaagacc atctccaagg ccaaggggca gccccgcgag 1200

ccacaggtgt acaccctgcc cccatcccgc gaggagatga ccaagaacca ggtgagcctg 1260

acctgcctgg tgaagggctt ctaccccagc gacatcgccg tggagtggga gagcaacggg 1320

cagcccgaga acaactacaa gaccaccccc cccgtgctgg actccgacgg ctccttcttc 1380
ctgtacagca agctgaccgt ggacaagagc aggtggcagc aggggaacgt gttctcctgc 1440
tccgtgatgc acgaggccct gcacaaccac tacacccaga agagcctctc cctgtccccc 1500
ggcaagtgat aagtcgacac ctgatc 1526